# EUROPEAN PATENT SPECIFICATION

(11) **EP 3 774 747 B1**
(45) Date of publication and mention of the grant of the patent: **13.11.2024**
(21) Application number: 19730915.6
(22) Date of filing: 04.04.2019
(51) Int. Cl.: C07D 265/38, G01N 33/52

(54) **BENZOPHENOXAZINE DERIVATIVES FOR DIAGNOSIS OF NEOPLASIA OR INFECTION**
BENZOPHENOXAZINE DERIVATE ZUR DIAGNOSE VON NEOPLASIE ODER INFEKTIONEN
DERIVES DE BENZOPHENOXAZINE POUR LA DIAGNOSE DU NEOPLASME OU DES INFECTIONS

(30) Priority: 04.04.2018 PT 2018110665
(43) Date of publication of application: 17.02.2021
(73) Proprietor: Universidade do Minho, 4704-553 Braga (PT)
(72) Inventor: DO SAMEIRO TORRES GONÇALVES, Maria, 4710-057 Braga (PT); PAIVA DA SILVA LEITÃO, Maria Inês, 2775-575 Cascais (PT); MARQUES FERREIRA DE SOUSA MOREIRA, Maria João, 4710-057 Braga (PT)
(74) Representative: Patentree
(86) International application number: PCT/IB2019/052779
(87) International publication number: WO 2019/193544

(56) References cited:
- WO-A1-01/18124
- BENHUA WANG ET AL: "A Nile blue based infrared fluorescent probe: imaging tumors that over-express cyclooxygenase-2", CHEMICAL COMMUNICATIONS, vol. 51, no. 4, 1 January 2015 (2015-01-01), UK, pages 792 - 795, XP055611863, ISSN: 1359-7345, DOI: 10.1039/C4CC08915D
- RAMA RAJU B ET AL: "Novel Nile Blue derivatives as fluorescent probes for DNA", DYES AND PIGMENTS, ELSEVIER APPLIED SCIENCE PUBLISHERS. BARKING, GB, vol. 99, no. 1, 15 May 2013 (2013-05-15), pages 220 - 227, XP028574322, ISSN: 0143-7208, DOI: 10.1016/J.DYEPIG.2013.05.007
- RAJU B RAMA ET AL: "Synthesis, photophysical characterisation and photostability studies of NIR probes with aliphatic, aromatic and chlorinated terminals in 5- and 9-amino positions of benzo[a]phenoxazines", DYES AND PIGMENTS, ELSEVIER APPLIED SCIENCE PUBLISHERS. BARKING, GB, vol. 132, 30 April 2016 (2016-04-30), pages 204 - 212, XP029566743, ISSN: 0143-7208, DOI: 10.1016/J.DYEPIG.2016.04.049
- RAJU B RAMA ET AL: "Synthesis of new benzo[a]phenoxazinium probes possessing carboxylic ester, hydroxyl and amino functional groups: Photophysical studies in dry ethanol and conjugation with CdTe quantum dots", DYES AND PIGMENTS, ELSEVIER APPLIED SCIENCE PUBLISHERS. BARKING, GB, vol. 110, 16 April 2014 (2014-04-16), pages 203 - 213, XP029039932, ISSN: 0143-7208, DOI: 10.1016/J.DYEPIG.2014.04.006
- RAJU B RAMA ET AL: "Synthesis and photophysical properties of side-chain chlorinated benzo[a]phenoxazinium chlorides", TETRAHEDRON, ELSEVIER SCIENCE PUBLISHERS, AMSTERDAM, NL, vol. 69, no. 11, 18 January 2013 (2013-01-18), pages 2451 - 2461, XP028975999, ISSN: 0040-4020, DOI: 10.1016/J.TET.2013.01.035
- MARIA LEITÃO ET AL: "Benzo[a]phenoxazinium chlorides functionalized with chloride atoms and/or ester groups", 1 November 2015 (2015-11-01), XP055611896, Retrieved from the Internet <URL:https://sciforum.net//manuscripts/3031/manuscript.pdf> DOI: 10.3390/ecsoc-19-a006
- VAN STAVEREN HUGO J ET AL: "Fluorescence Imaging and Spectroscopy of Ethyl Nile Blue A in AnimalModels of (Pre)malignancies", PHOTOCHEMISTRY AND PHOTOBIOLOGY 2011 JUL-AUG,, vol. 73, no. 1, 31 December 2000 (2000-12-31), pages 32 - 38, XP009515082, ISSN: 1751-1097, DOI: 10.1562/0031-8655(2001)0730032FIASOE2.0.CO2

## Description

### TECHNICAL DOMAIN

The present disclosure relates to a new compound for cell coloration, fixation and staining, and preparation method and use. For both laboratorial and clinical practices, its aim is cell preservation and coloration, within the scope of health sciences. This new compound can be used clinical and laboratorial diagnosis, with high applicability in screening campaigns, targeting several health care professionals with different backgrounds.

### BACKGROUND

Early diagnosis of several diseases that allows timely intervention and management planning is largely dependent on efficient and easily accessible methods of detection. In routine histochemistry, gynecological or non-gynecologic cytologies, schedules currently available for processing tissues and staining sections consist in various phases, require diverse specific equipment, several laboratory techniques and hours of work for completion. The fixation and staining are different steps of currently screening process, since they are incompatible. The fixators act only as preservatives of the cells, whereas the stains promote the differentiation between the cell organelles. The mentioned laboratory techniques are not feasible to be performed by clinicians (such as doctors or dentists).

Recently, PCT/IB2012/057799 described a single composition with both a fixator (ethanol) and a stain (toluidine blue). It consists in an ethanoic solution of toluidine blue, a metachromatic stain, which stains differently basophilic and acidophilic structures. However, this solution presents some limitations, such as cell staining amplitude and high amounts of dye in the compositions used for this purpose.

The need to early detect diseases in a noninvasive and simple manner using a low cost diagnostic method performed *in situ* and in a short time is one of the biggest challenges in medicine today. The easy detection of anomalies in a gynecological appointment after collection of a sample of cells without the need to wait several days for the result of a Papanicolaou test or in a visit to the dentist with cells of only one smear is extremely useful in clinical practice with implications in People's health.

The present disclosure describes compounds, the preparation method and uses with very promising clinical applications for diagnostic purposes in odontology, gynecological and non-gynecological fields.

The diagnosis can be made during the medical appointment in doctors' offices, by professionals with diverse backgrounds (e.g., dentists, gynecologists, among others). The method does not need specific conditions for its accomplishment; it is easy to perform, fast in the realization as well as in the presentation of results.

The composition that was now developed presents some advantages regarding the state of the art, such as the simultaneous fixation and staining of cells, the staining of various cell types (e.g. mucous cells, like cells from the oral cavity and, cells from exo and endocervix), the use of a lower concentration of dye in the formulations and a reduction in staining time and faster results.

Due to all the mentioned facts, the developed solution conciliates and potentiates the chemical characteristics of the involved products, without losing the practical side of its application.

It allows the diagnosis of several cellular types, with high quality, structure differentiation and with a fast execution time. This way, the described composition in the present disclosure allows simultaneously fixing and staining a cytological sample.

### GENERAL DESCRIPTION

The current fixative and tissue processing techniques developed so far include several processing stages, which force several laboratorial steps, are time consuming and unfeasible to be executed by low experienced professionals or that are not familiarized with the laboratory processing. For example, the referred laboratory techniques are not feasible to be executed by clinicians (doctors or dentists). Nowadays, one of the biggest challenges in medicine is the early diagnosis of diseases so as to allow healing or less aggressive treatments that results in high quality of life as long as possible. The development of noninvasive and simple detection procedures with low cost that can be executed in a simple way and allow rapid results are of great importance.

Over the years a number of methodologies based on detection of cellular anomalies by morphological analysis using fluorescent and non-fluorescent organic dyes as stains have been reported (Atale N., Guptas S., Yadav U. C. S., Rani V., "Cell-death assessment by fluorescent and nonfluorescent cytosolic and nuclear staining techniques", J. Microsc. 2014, 255, 7-19). Optical imaging is preferred over other nuclear based techniques as it is inexpensive, exhibits high availability and option of multi-target tracing both *in vivo* and *in vitro.* An ideal dye for biological targets should display suitable wavelength of absorption and emission, preferably within the near infrared (NIR) range (650-900 nm), with minimum photodamage, and high affinity for specific labelling of an organelle or molecule.

Owing to the strong absorption and NIR fluorescent emission wavelengths, benzo[*a*]phenoxazines are interesting compounds for biological applications. Moreover, noncovalent bonding is a common feature of benzo[*a*]phenoxazinium dyes that allows their use for staining purposes in gel electrophoresis and study of their interactions (intercalative and/or groove binding) with DNA (Raju B. R., Naik S., Coutinho P. J. G., Goncalves M. S. T., "Novel Nile Blue derivatives as fluorescent probes for DNA", Dyes Pigm. 2013, 99, 220-227). These compounds have also been studied as lysosome trackers (Liu W., Sun R., Ge J. F., Xu Y. J., Xu Y., Lu J. M., Itoh I., Ihara M., "Reversible near-infrared pH probes based on benzo[a]phenoxazine", Anal. Chem. 2013, 85, 7419-7425), sensors for reversible pH measurements and NIR live bioimaging purposes (Madsen J., Canton I., Warren N. J., Themistou E., Blanazs A., Ustbas B., Tian X., Pearson R., Battaglia G., Lewis A. L., Armes, S. P. "Nile Blue-based nanosized pH sensors for simultaneous far-red and near-infrared live bioimaging", J. Am. Chem. Soc. 2013, 135, 14863-14870). Nile Blue dye, the most widely known benzo[*a*]phenoxazine, is a fluorochromophore used routinely in histology to impart a blue color to cell nuclei, where it highlights the distinction between neutral lipids (triglycerides, etc.), which are stained pink and fatty acids, which are stained blue.

Additionally, benzo[*a*]phenoxazine derivatives are interesting as antifungals (Frade V. H. J., Sousa M. J., Moura J. C. V. P., Goncalves M. S. T., "Synthesis of naphtho[2,3-a]phenoxazinium chlorides. Structure-activity relationships of these heterocycles and benzo[a]phenoxazinium chlorides as new antimicrobials", Bioorg. Med. Chem. 2008, 16, 3274-3282; Frade V. H. J., Sousa M. J., Moura J. C. V. P., Goncalves M. S. T., "Synthesis, characterisation and antimicrobial activity of new benzo[a]phenoxazine based fluorophores", Tetrahedron Lett. 2007, 48, 8347-8352), antimalarials (Mizukawa Y., Ge J-F., Md A. B., Itoh I., Scheurer C., Wittlin S., Brun R., Matsuoka H., Ihara M., "Novel synthetic route for antimalarial benzo[a]phenoxazine derivative SSJ-183 and two active metabolites", Bioorg. Med. Chem. 2014, 22, 3749-3752), as well as photosensitizers in photodynamic therapy (PDT) for treatment of *Candida albicans* biofilms (Lopes M., Alves C. T., Raju B. R., Goncalves M. S. T., Coutinho P. J. G., Henriques M., Belo I., "Application of benzo[a]phenoxazinium chlorides in antimicrobial photodynamic therapy of Candida albicans biofilms", J. Photochem. Photobiol: B 2014, 141, 93-99).

The present disclosure is directed to benzo[*a*]phenoxazine derivatives for use in a method of diagnosis of cancer or neoplasia in a sample having the following structural formula I: 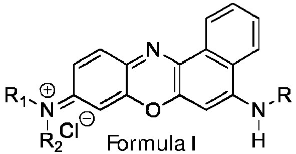
or another salt, solvate, hydrate, tautomer, stereoisomer,
wherein R, R₁, R₂, are independently selected from each other;
R is a propyl group or an isopropyl group or an isopentyl group,
R₁ and R₂ selected from hydrogen, C₃-C₈ unsubstituted alkyl chain or (CH₂)₃NH₂.HBr.

In the present disclosure, the term "alkyl" relates to a linear or cyclic hydrocarbon group comprising from 1 to 20 carbon atoms, preferably 1 to 8 carbon atoms, preferably from 1 to 3 carbon atoms. Among alkyl groups it can be selected from the methyl, ethyl, n-propyl, isopropyl, butyl, pentyl, hexyl and hepthyl groups.

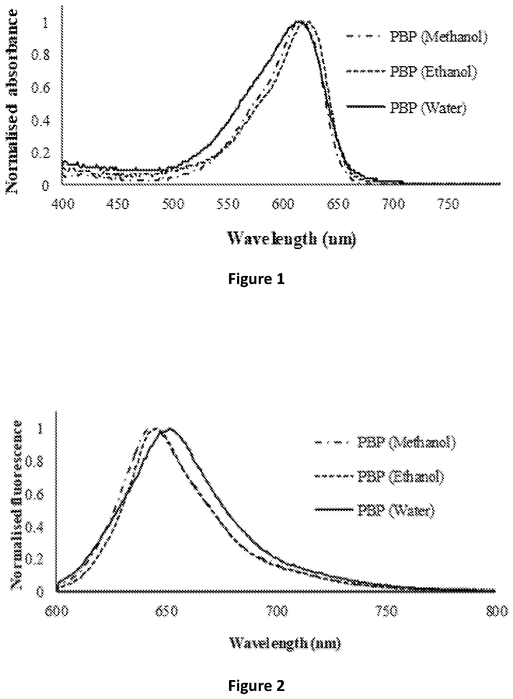

The present disclosure describes benzo[*a*]phenoxazine derivatives, displaying good photophysical properties such as high molar extinction coefficients, good fluorescence quantum yields and solubility in water, with capability to act simultaneously as fixative and stain in a composition for cytology, preferably mucous cells, like cells from the oral cavity and, cells from exo and endocervix (Figures 1-3). Most preferably, the present disclosure includes the *N*-(5-(propylamino)-9*H*-benzo[*a*]phenoxazin-9-ylidene)propan-1-aminium chloride **PBP,** Formula II or 3-amino-*N*-(5-(isopentylamino)-9*H*-benzo[*a*]phenoxazin-9-ylidene)propan-1-aminium chloride hydrobromide **ABP,** Formula III.

The fluorochromophore PBP comprises the formula I, wherein R = R₁ = CH₂CH₂CH₃, R₂ = H.

The fluorochromophore **ABP** comprises formula I, wherein R = CH₂CH₂CH(CH₃)₂, R₁ = CH₂CH₂CH₂NH₂.HBr, R₂ = H. 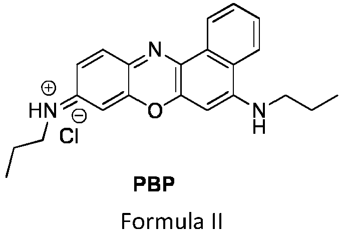 or

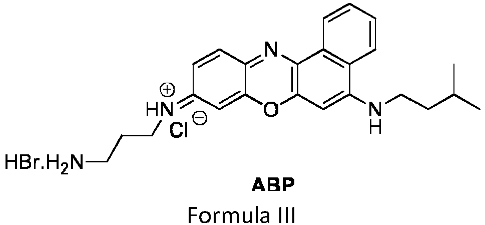

The present disclosure also describes a composition comprising the compound formerly described; preferably compound **PBP** or **ABP,** in a suitable concentration for practical uses (0.8-1.5 mM, preferably 0.92 mM). This composition is suitable for medical use, such as in neoplasic detection, preferably cervix and oral cavity cancers.

The use of this composition permits a significant simplification in the procedure, reduction of the time of realization and costs. Simultaneously, the application of **PBP** or **ABP** to cytology is innovative, either gynecologic or non-gynecologic, since **PBP** or **ABP** has never been described.

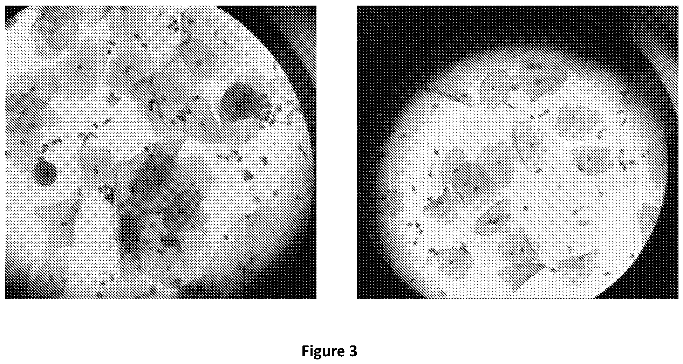

The fixative and staining composition described in the present disclosure allows, less than 1 minute, having a sample ready for microscopic observation. It provides 100% cell colouring (Figure 3 and Table 2).

The fixative and staining composition described in the present disclosure is based in an alcoholic solution of **PBP** or **ABP.** The alcohols used are ethanol, isopropanol, methanol, water or their mixtures, in which **PBP** or **ABP** completely dissolves, but preferentially ethanol that commonly is used as medicinal solvent and is presented in liquid preparations of medicine. The composition will be part of a kit that in a clinical environment the professionals will have as a portable test, and will allow the evaluation of potentially serious pathologies in their patients with results in a short time. The current procedure that implies sending the sample to other places, to well-equipped laboratories, which sometimes compromised or made the diagnosis impossible, will be circumventing with the present disclosure. The cost associated to cytology will also be drastically reduced.

The present disclosure allows the morphological identification of cellular structures, namely, the nucleus, the cytoplasm and their membranes. The morphological details are evidenced through different tonalities, differentiating the nucleus by a more intense blue of the cytoplasm. In addition, with the coloring properties of the proposed composition it is possible to clearly identify the characteristic changes of atypical or malignant cells distinguishing them from the other normal cells.

The new composition described is efficient, simple, fast and an economic approach for all process. It becomes possible to achieve a screening or diagnosis in a simple way anywhere in the world with the final benefit to people and health in general.

The present disclosure also describes the method for obtaining a fixative, preservative and staining composition for cells, which consists in the solubilisation of **PBP** powder or **ABP** powder in a solvent that can be water or an alcohol, or their mixture, with stirring, at room temperature (23°C).

The starting materials and the precursors used in the synthesis of dyes with formula I in the present disclosure can be commercially available, or prepared according to procedures described in the literature (Leitão M. I. P. S., Raju B. R., Naik S., Coutinho P. J. G., Sousa M. J., Gonçalves M. S. T., "Synthesis and photophysical studies of new benzo[a]phenoxazinium chlorides as potential antifungal agents", Tetrahedron Lett. 2016, 57, 3936-3941; Alves C. M. A., Naik S., Coutinho P. J. G., Gonçalves M. S. T., "Novel long alkyl side-chain benzo[a]phenoxazinium chlorides: synthesis, photophysical behaviour and DNA interaction", Tetrahedron, 2009, 65, 10441-10452), and presented in this disclosure.

In an embodiment the structural formula of the compound is 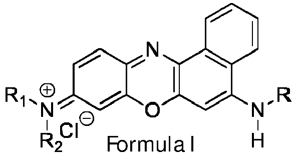
or another salt, solvate, hydrate, tautomer, stereoisomer,
wherein R, R₁, R₂, are independently selected from each other;
R is a propyl group or an isopropyl group or an isopentyl group;
R₁ and R₂ selected from hydrogen, C₃-C₈ unsubstituted alkyl chain or (CH₂)₃NH₂.HBr;
for use in a method of diagnosis of cancer or neoplasia in a sample.

In an embodiment, R₁ or R₂ may be a C₃-C₈ unsubstituted alkyl chain, more preferably wherein R₁ or R₂ may be a C₃ unsubstituted alkyl chain.

In an embodiment, R₂ may be hydrogen.

In an embodiment R is a propyl group or an isopropyl group or an isopenthyl group.

In an embodiment R is a propyl group or an isopenthyl group.

In an embodiment R₁ is propyl or an isopropyl group, preferably R₁ may be propyl.

In an embodiment R₁ is a propyl.

In an embodiment, R₁ or R₂ may be (CH₂)₃NH₂HBr.

In an embodiment R₂ is hydrogen.

An aspect of the present disclosure relates to the compound of the following structures:

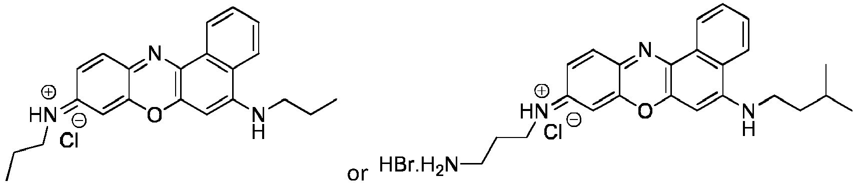

The present disclosure also relates to a compound as described herein for use in medicine.

The present disclosure also relates to a compound as described herein for use in a method of diagnosis of a neoplasia or infection in a sample.

In an embodiment, the neoplasia is a cervix cancer or oral cavity cancer.

In an embodiment, a composition for use in cell staining and fixation comprises the compound and a suitable solvent.

In an embodiment, the quantity of compound in the composition is less than 0.1% (m/v), varying between 0.02 - 0.1% (m/v).

In an embodiment, the quantity of compound in the composition is 0.02 - 0.075% (m/v).

In an embodiment, the quantity of compound in the composition is 0.03 - 0.04% (m/v).

In an embodiment, the solvent is selected between water, alcohol base or their mixtures.

In an embodiment the alcohol base is selected between ethanol, methanol, isopropanol, or mixtures thereof.

In an embodiment the alcohol base is preferably ethanol.

This disclosure also relates to a composition comprising any of the compounds described herein, preferably for use in cells colouring and staining.

This compound and composition may be used in medicine, for diagnostic of a neoplasia or infection. Is used for the diagnostic of cancer like cervix or oral cavity cancer.

### BRIEF DESCRIPTION OF THE DRAWINGS

**Figure 1** shows the normalised absorption spectra of **PBP** in ethanol, methanol and water.
**Figure 2** shows the normalised fluorescence emission spectra of **PBP** in ethanol, methanol and water.
**Figure 3** shows the optical microscopic images of cervix cells with the fixative and staining composition described in the present disclosure.

### DETAILED DESCRIPTION

The present disclosure relates to new benzo[*a*]phenoxazine derivatives for use in a method of diagnosis of cancer or neoplasia in a sample, fluorochromophores having the following structural formula I: 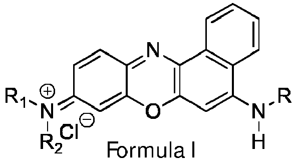 wherein R is a propyl group or an isopropyl group or an isopentyl group and R₁ and R₂ are hydrogen, a C₃-C₈ unsubstituted alkyl chain or (CH₂)₃NH₂.HBr.

The present disclosure also includes the fluorochromophore **PBP** or the fluorochromophore **ABP.**

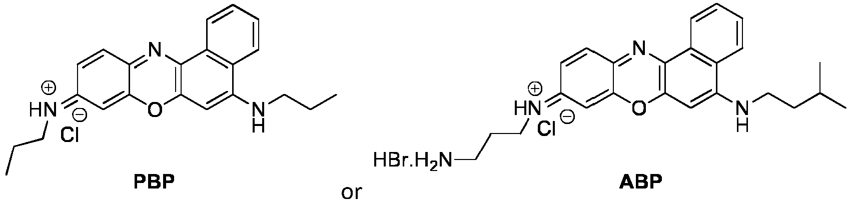

The starting materials used in the present disclosure can be commercially available, and the precursors (**1, 3, 5** and **8**) can be prepared according to the procedures described in the literature (Leitão M. I. P. S., Raju B. R., Naik S., Coutinho P. J. G., Sousa M. J., Goncalves M. S. T., "Synthesis and photophysical studies of new benzo[a]phenoxazinium chlorides as potential antifungal agents", Tetrahedron Lett. 2016, 57, 3936-3941; Alves C. M. A., Naik S., Coutinho P. J. G., Gonçalves M. S. T., "Novel long alkyl side-chain benzo[a]phenoxazinium chlorides: synthesis, photophysical behaviour and DNA interaction", Tetrahedron, 2009, 65, 10441-10452; Raju B. R., Sampaio D. M. F., Silva M. M., Coutinho P. J. G., Gonçalves M. S. T. "Ultrasound promoted synthesis of Nile Blue derivatives", Ultrason. Sonochem., 2014, 21, 360-366), and presented in this disclosure. Data related to material and instruments used are mentioned at this point.

### Chemical procedures for the synthesis of the above-mentioned compounds, in particular fluorochromophore PBP and ABP

The synthesis of **PBP** and possible procedures for the preparation of the required precursors, as well as the fundamental spectroscopic characterization are described herein.

Synthesis of 3-(propylamino)phenol **1** and 3-(dipropylamino)phenol **2** (Leitão M. I. P. S., Raju B. R., Naik S., Coutinho P. J. G., Sousa M. J., Gonçalves M. S. T., "Synthesis and photophysical studies of new benzo[a]phenoxazinium chlorides as potential antifungal agents", Tetrahedron Lett. 2016, 57, 3936-3941).

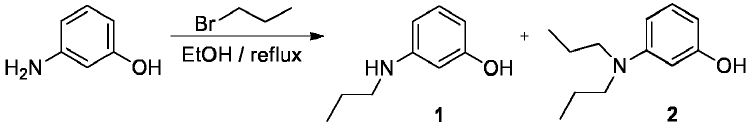

In an embodiment, the procedure for the preparation of 3-(propylamino)phenol **1** and 3-(dipropylamino)phenol **2** is as follows: to a suspension of 3-aminophenol (2.0 g, 18.3 mmol) in ethanol (8 mL), 1-bromopropane (2.50 mL, 27.5 mmol) was added, and the reaction was refluxed during 4h 30 min, followed by TLC (dichloromethane / methanol 9.5:0.5). After solvent evaporation and chromatography with dichloromethane and dichloromethane / methanol, mixtures of increasing polarity, as the eluent, 3-(propylamino)phenol **1** was isolated as brown liquid (1.410 g, 51%), TLC (dichloromethane): *R_{f}* = 0.17. FTIR (KBr 1%): *v*ₘₐₓ 3250, 2642, 2500, 2415, 1619, 1577, 1487, 1426 cm⁻¹. ¹H NMR (400 MHz, CDCl₃): *δ* 1.05 (t, *J* = 7.2 Hz, 3 H, NHCH₂CH₂*CH₃*), 1.75-1.86 (m, 2 H, NHCH₂*CH₂*CH₃), 3.35-3.46 (m, 2 H, NH*CH₂*CH₂CH₃), 6.92-7.01 (m, 3 H, 4-H, 6-H, 2-H), 7.35-7.42 (m, 1 H, 5-H) ppm. ¹³C NMR (100.6 MHz, CDCl₃): *δ* 11.14 (NHCH₂CH₂*CH₃*), 20.37 (NHCH₂*CH₂*CH₃), 54.75 (NH*CH₂*CH₂CH₃), 110.58 (C-2), 114.09 (C-6), 117.63 (C-4), 132.03 (C-5), 137.52 (C-3), 160.19 (C-1) ppm. HRMS (TOF El): found [M⁺] 151.1003; C₉H₁₃NO requires [M⁺] 151.0997. The 3-(dipropylamino)phenol **2** was also obtained as a brown solid (0.715 g, 21%). Mp = 99.7-100.3 °C. TLC (dichloromethane): *R_{f}* = 0.22. FTIR (KBr 1%): *v*ₘₐₓ 3208, 3188, 3053, 2966, 2880, 2711, 2672, 2636, 2508, 1613, 1508, 1488, 1471, 1456, 1431, 1417 cm⁻¹. ¹H NMR (400 MHz, CDCl₃): *δ* 0.84 (t, *J* = 7.6 Hz, 6 H, N(CH₂CH₂*CH₃*)₂), 1.59 (br s, 4 H, N(CH₂*CH₂*CH₃)₂), 3.30 (t, *J* = 8.0 Hz, 4 H, N(*CH₂*CH₂CH₃)₂), 6.87 (br s, 2 H, 4-H, 6-H), 7.05 (br s, 1 H, 2-H), 7.17 (t, *J* = 8.0 Hz, 1 H, 5-H) ppm. ¹³C NMR (100.6 MHz, CDCl₃): *δ* 10.85 (N(CH₂CH₂*CH₃*)₂), 18.86 (N(CH₂*CH₂*CH₃)₂), 58.17 (N(*CH₂*CH₂CH₃)₂), 107.61 (C-2), 113.60 (C-4, C-6), 130.63 (C-5), 137.52 (C-3), 158.22 (C-1) ppm. HRMS (TOF El): Found [M⁺] 193.1474; C₁₂H₁₉NO requires [M⁺] 193.1467.

### Synthesis of 2-nitroso-5-(propylamino)phenol hydrochloride 3

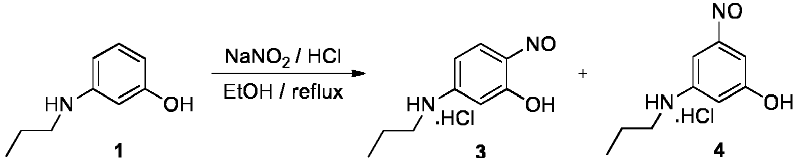

In an embodiment, the procedure for the preparation of 2-nitroso-5-(propylamino)phenol hydrochloride **3** was as follows: to an ice-cold solution of 3-(propylamino)phenol **3** (0.302 g, 2 mmol) in ethanol (4 mL), 2 M hydrochloric acid (0.8 mL) was added and stirred during 5 min. The solution of sodium nitrite (0.166 g, 2.4 mmol) in water (0.6 mL) was then added dropwise within an interval of 10-15 min. The resulting mixture was stirred for 3 h and monitored by TLC (dichloromethane / methanol, 9.5:0:5). After evaporation of the reaction, green solid (0.468 g) having 2-nitroso-5-(propylamino)phenol hydrochloride **3** and its isomer 3-nitroso-5-(propylamino)phenol hydrochloride **4** (3:2) were obtained and used in the following step without any purification.

### Synthesis of N-propylnaphthalen-1-amine 5

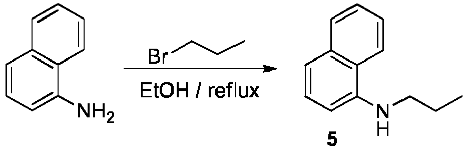

Procedure for the preparation of *N*-propylnaphthalen-1-amine **5** (Alves C. M. A., Naik S., Coutinho P. J. G., Gonçalves M. S. T., "Novel long alkyl side-chain benzo[a]phenoxazinium chlorides: synthesis, photophysical behaviour and DNA interaction", Tetrahedron 2009, 65, 10441-10452).

In an embodiment, to a solution of 1-naphthylamine (2.0 g, 14 mmol) in ethanol (2 mL), 1-bromopropane (1.81 mL; 20 mmol) was added and the resulting mixture was refluxed for 18 h, and monitored by TLC (chloroform / *n*-hexane, 5:2). After evaporation of the solvent and dry chromatography purification on silica gel, with chloroform / *n*-hexane, mixtures of increasing polarity, as the eluent, compound **5** was obtained as dark brown oil (1.55 g, 60%). TLC (chloroform / *n*-hexane, 5:2): *R*_{f} = 0.55. ¹H NMR (300 MHz, CDCl₃): *δ* 1.09 (t, *J* = 7.8 Hz, 3 H, CH₃), 1.80-1.90 (m, 2 H, NHCH₂*CH₂*), 3.29 (t, *J* = 7.2 Hz, 2 H, NH*CH₂*CH₂), 6.75 (d, *J* = 7.2 Hz, 1 H, 4-H), 7.29 (d, *J* = 8.1 Hz, 1 H, 2-H), 7.38 (t, *J* = 7.8 Hz, 1 H, 3-H), 7.42-7.50 (m, 2 H, 6-H and 7-H), 7.78-7.82 (m, 1 H, 8-H), 7.84-7.90 (m, 1 H, 5-H) ppm. ¹³C NMR (75.4 MHz, CDCl₃): *<5* 11.75 (CH₃), 22.33 (NHCH₂*CH₂*), 46.65 (NHCH₂*CH₂*), 105.62 (C-4), 118.03 (C-2), 119.89 (C-5), 123.51 (C-4a), 124.83 (C-7), 125.73 (C-6), 126.52 (C-3), 128.66 (C-8), 134.30 (C-8a), 142.65 (C-1) ppm. FTIR (neat): *v*ₘₐₓ 3427, 2958, 2923, 2852, 1624, 1583, 1475, 1463, 1455, 1409, 1379, 1345, 1280, 1142, 1080, 1016, 975, 858, 798, 768, 666 cm⁻¹. HRMS (TOF El): found [M⁺] 186.12773; C₁₃H₁₆N [M⁺] requires 186.12823.

### Synthesis of fluorochromophore PBP

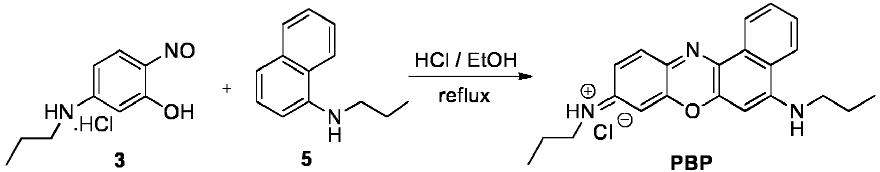

In an embodiment, the procedure for the preparation of **PBP** is as follows. To a cold solution (ice bath) of nitrosophenol hydrochloride **3** (0.360 g, 1.0 mmol to 0.540 g, 1.5 mmol, preferably 0.396 g, 1.1 mmol) in methanol or preferentially ethanol (2 mL), *N*-propylnaphthalen-1-amine **5** (0.186 g, 1.0 mmol to 0.279 g, 1.5 mmol, preferably 0.205 g, 1.1 mmol) and concentrated hydrochloride acid (0 to 0,10 mL, preferably 0.027 mL) were added. The reaction mixture was refluxed for 8 h and monitored by TLC (dichloromethane / methanol 9.5:0.5). After evaporation of the solvent and column chromatography purification on silica gel with dichloromethane and dichloromethane / methanol, mixtures of increasing polarity, as the eluent, *N*-(5-(propylamino)-9*H*-benzo[*a*]phenoxazin-9-ylidene)propan-1-aminium **PBP** was obtained as a blue solid (0.050 g, 13%). R*_{f}* = 0.40 (dichloromethane / methanol, 9:1). Mp = 223-225°C. FTIR (KBr 1%): *v*ₘₐₓ 3426, 3202, 1641, 1589, 1548, 1496, 1454, 1434, 1384, 1324, 1284, 1235, 1187, 1160, 1120, 1002, 950, 819, 773 cm⁻¹. ¹H NMR (400 MHz, CD₃OD): *δ* 1.08-1.16 (m, 6 H, 2×NHCH₂CH₂*CH₃*), 1.74-1.83 (m, 2 H, NHCH₂CH₂CH₃), 1.86-1.95 (m, 2 H, NHCH₂*CH₂*CH₃), 3.31-3.34 (m, 2H, NH*CH₂*CH₂CH₃), 3.65 (t, *J* = 7.2 Hz, 2 H, NHCH₂CH₂CH₃), 6.65 (d, *J* = 2.0 Hz, 1 H, 8-H), 6.82 (s, 1 H, 6-H), 7.04 (dd, *J* = 9.2 and 1.6 Hz, 1 H, 10-H), 7.65 (d, *J* = 8.8 Hz, 1 H, 11-H), 7.75 (t, *J* = 7.2 Hz, 1 H, 3-H), 7.85 (t, *J* = 7.2 Hz, 1 H, 2-H), 8.26 (d, *J* = 8.4 Hz, 1 H, 4-H), 8.71 (d, *J* = 8.0 Hz, 1 H, 1-H) ppm. ¹³C NMR (100.6 MHz, CD₃OD): *δ* 11.77 (NHCH₂CH₂*CH₃*), 11.84 (NHCH₂CH₂*CH₃*), 23.02 (NHCH₂*CH₂*CH₃), 46.44 (NH*CH₂*CH₂CH₃), 47.38 (NH*CH₂*CH₂CH₃), 94.19 (C-6), 95.36 (C-8), 119.43 (C-10), 123.76 (C-4), 124.59 (Ar-C), 125.41 (C-1), 130.79 (C-3), 131.99 (Ar-C), 132.39 (ArC), 132.80 (C-2), 133.89 (C-11), 134.21 (Ar-C), 150.19 (Ar-C), 152.84 (Ar-C), 158.12 (C-9), 159.04 (C-5) ppm. HRMS (FAB): found [M⁺] 388.23834; C₂₅H₃₀N₃O requires [M⁺]: 388.23774.

In an embodiment, the absorption and fluorescent emission of PBP in different solvents was determined. Electronic absorption and emission spectra of 10⁻⁶ and 10⁻⁷ M solutions in ethanol, methanol and water were measured for benzo[*a*]phenoxazinium chloride **PBP** (**Table 1,** **Figures 1** and **2**). The relative fluorescence quantum yields (*Φ*_{F}) were determined using Oxazine 1 as a standard (*Φ*_{F} = 0.11 in ethanol) at 590 nm excitation.

The absorption maxima (λ_{abs}) for compound **PBP** in ethanol is 622 nm, whereas in methanol and water λ_{abs} is 617 nm, with molar extinction coefficients (*ε*) higher, log *ε* in the range 4.96 to 5.08, being the lowest value in water. The emission maxima (λₑₘ) was found to be in the range of 643 and 651 nm at excitation of 590 nm with moderate Stokes shifts (Δλ, 23 and 34 nm). As a result, the solvent change does not significantly modify the λ_{abs} values of **PBP,** with a hypochromic shift of 5 nm from ethanol to methanol and water. Comparison of the emission wavelength values in ethanol, methanol and water reveal a bathochromic shift of 6 nm when changing from alcohol to aqueous solutions. Relative fluorescence quantum yields in ethanol and methanol are higher (0.28) than in water (0.07).

### Procedure for the preparation of ABP

### Synthesis of 3-((3-aminopropyl)amino)phenol hydrobromide 6

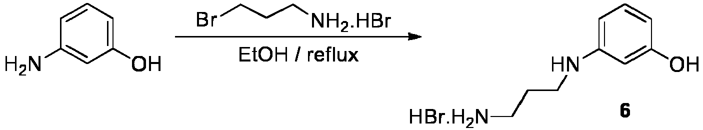

In an embodiment, the procedure for the preparation of 3-((3-aminopropyl)amino)phenol hydrobromide **6** was as follows. To a suspension of 3-aminophenol (0.500 g, 4.59 mmol), in ethanol (4 mL), 3-bromopropan-1-amine hydrobromide (1.250 g, 5.50 mmol) was added, and the reaction was refluxed during 8 h, followed by TLC (dichloromethane / methanol, 9:1). After solvent evaporation and chromatography with dichloromethane and dichloromethane / methanol, mixtures of increasing polarity, as the eluent, 3-((3-aminopropyl)amino)phenol hydrobromide **6** was isolated as a white oily solid (0.86 g, 58%), TLC (dichloromethane / methanol, 9:1): *R*_{f} = 0.65. ¹H NMR (300 MHz, DMSO-*d₆*): *δ* 1.80-2.00 (m, 2 H, NHCH₂*CH₂CH₂*NH₂), 2.78-3.01 (m, 2 H, NHCH₂CH₂*CH₂*NH₂), 3.27 (t, *J* = 7.2 Hz, 2 H, NH*CH₂*CH₂CH₂NH₂), 6.50-6.80 (m, 3 H, H-4, H-2 and H-6), 7.20 (t, *J* = 8.1 Hz, 1 H, H-5), 7.81 (broad s, 3 H, NH₂·HBr), 9.70 (broad s, 1 H, OH) ppm.

### Synthesis of 5-((3-aminopropyl)amino)-2-nitrosophenol hydrobromide hydrochloride 7

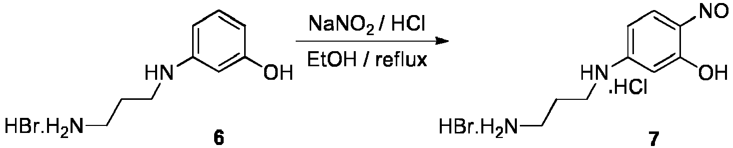

In an embodiment, the procedure for the preparation of 5-((3-aminopropyl)amino)-2-nitrosophenol hydrobromide hydrochloride **7** was carried out as follows. To an ice-cold solution of 3-((3-aminopropyl)amino)phenol hydrobromide **6** (0.100 g, 0.40 mmol) in ethanol (1 mL), 12 M hydrochloric acid (1 mL) was added and stirred during 5 min. The solution of sodium nitrite (0.031 g, 0.45 mmol) in water (1 mL) was then added dropwise within an interval of 10-15 min. The resulting mixture was stirred for 3 h and monitored by TLC (dichloromethane / methanol, 9:1). After evaporation of the reaction, a green solid (0.094 g) having 5-((3-aminopropyl)amino)-2-nitrosophenol hydrobromide hydrochloride **7** was obtained and used in the following step without any purification.

Synthesis of *N*-isopenthylnaphthalen-1-amine **8** (Raju B. R., Sampaio D. M. F., Silva M. M., Coutinho P. J. G., Gonçalves M. S. T. "Ultrasound promoted synthesis of Nile Blue derivatives", Ultrason. Sonochem., 2014, 21, 360-366).

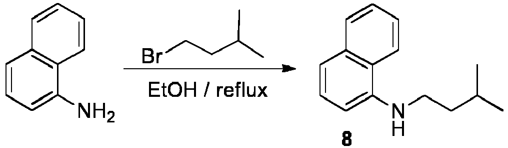

In an embodiment, the procedure for the preparation of *N*-isopentylnaphthalen-1-amine **8** was as follows. To a solution of 1-naphthylamine (0.500 g, 3.49 mmol) in ethanol (3 mL), 1-bromo-3-methylbutane (0.581 g, 3.84 mmol) was added and the resulting mixture was refluxed for 14 h, and monitored by TLC (dichloromethane / methanol 9:1). After evaporation of the solvent and column chromatography purification on silica gel, with dichloromethane and dichloromethane / methanol 99:1, as the eluent, compound **8** was obtained as dark brown oil (0.401 g, 54%). TLC (dichloromethane / methanol, 9.9:0.1): *R*_{f} = 0.54. ¹H NMR (400 MHz, CDCl₃): *δ* 1.00 (d, *J* = 6.4 Hz, 6 H, 2×NHCH₂CH₂CH(*CH₃*)*₂*), 1.70-1.86 (m, 3 H, NHCH₂CH₂*CH*(CH₃)₂ and NHCH₂*CH₂*CH(CH₃)₂), 3.39 (t, *J* = 7.2 Hz, 2 H, NH*CH₂*CH₂CH(CH₃)₂), 3.50 (br s, 1 H, NH), 7.05 (dd, *J* = 6.2 and 2.4 Hz, 1 H, 2-H), 7.40-7.46 (m, 2 H, 3-H and 4-H), 7.49-7.56 (m, 2 H, 7-H and 6-H), 7.85 (dd, *J* = 7.0 and 2.0 Hz, 1 H, 5-H), 8.02-8.08 (dd, *J* = 7.2 and 2.0 Hz, 1 H, 8-H) ppm. ¹³C NMR (100.6 MHz, CDCl₃): *δ* 22.3 (NHCH₂CH₂CH(*CH₃*)*₂*), 25.9 (NHCH₂CH₂*CH*(CH₃)₂), 36.9 (NHCH₂*CH₂*CH(CH₃)₂), 44.5 (NH*CH₂*CH₂CH(CH₃)₂), 108.8 (C-2), 120.1 (C-3), 120.3 (C-8), 123.8 (C-4a), 125.2 (C-7), 125.8 (C-6), 126.0 (C-4), 128.4 (C-5), 134.1 (C-8a), 140.0 (C-1) ppm. FTIR (KBr 1%): *v*ₘₐₓ 3424, 3049, 2926, 2848, 2823, 1623, 1581, 1527, 1471, 1445, 1410, 1383, 1345, 1324, 1289, 1264, 1174, 1143, 1121, 1099, 1033, 784, 765 cm⁻¹. HRMS (El): found [M⁺] 213.1519; C₁₅H₁₉N [M⁺] requires 213.1517.

### Synthesis of fluorochromophore ABP

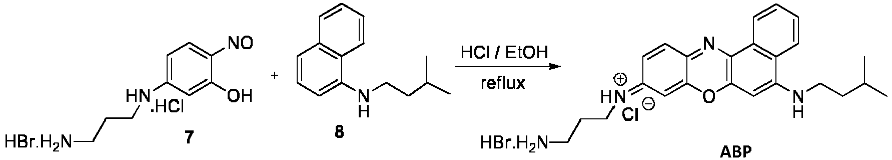

In an embodiment, the procedure for the preparation of **ABP** was as follows. To a cold solution (ice bath) of nitrosophenol hydrochloride **7** (0.0687 g, 2.22 mmol to 0.103 g, 0.33 mmol, preferably 0.076 g, 0.24 mmol) in methanol or preferentially ethanol (2 mL), *N-*isopenthylnaphthalen-1-amine **8** (0.047 g, 0.22 mmol to 0.070 g, 0.33 mmol, preferably 0.052 g, 0.24 mmol) and concentrated hydrochloride acid (0 to 0.10 mL, preferably 7.0×10⁻³ mL) were added. The reaction mixture was refluxed for 4 h and monitored by TLC (dichloromethane / methanol, 9:1). After evaporation of the solvent and column chromatography purification on silica gel with dichloromethane and dichloromethane / methanol, mixtures of increasing polarity, as the eluent, 3-amino-*N*-(5-(isopentylamino)-9*H*-benzo[*a*]phenoxazin-9-ylidene)propan-1-aminium chloride hydrobromide **ABP** was obtained as a blue solid (0.692 g, 43%). R*_{f}* = 0.90 (dichloromethane / methanol, 9:1). Mp = 223-225°C. FTIR (KBr 1%): *v*ₘₐₓ 3436, 2927, 1641, 1596, 1548, 1458, 1326, 1223, 1122, 1090, 778 cm⁻¹. ¹H NMR (400 MHz, CD₃OD): *δ* 1.05-1.13 (m, 6 H, 2×CH₃), 1.70-1.91 (m, 3 H, NHCH₂*CH₂*CH(CH₃)₂ and NHCH₂CH₂*CH*(CH₃)₂), 2.09-2.19 (m, 2 H, NHCH₂*CH₂CH₂*NH₂), 3.15 (t, *J* = 8.0 Hz, 2 H, NHCH₂CH₂*CH₂*NH₂), 3.56 (t, *J* = 6.8 Hz, 2 H, NH*CH₂*CH₂CH(CH₃)₂), 3.81 (t, *J* = 7.2 Hz, 2 H, NH*CH₂*CH₂CH₂NH₂), 6.93 (d, *J* = 2.4 Hz, 1 H, H-8), 7.03 (s, 1 H, H-6), 7.18 (dd, *J* = 9.2 and 2.4 Hz, 1 H, H-10), 6.90-7.04 (m, 2 H, H-3 and H-11), 7.98 (t, *J* = 7.2 Hz, 1 H, H-2), 8.42 (d, *J* = 8.0 Hz, 1 H, H-4), 8.98 (d, *J* = 7.6 Hz, 1 H, H-1) ppm. ¹³C NMR

(100.6 MHz, CD₃OD): *δ* 22.99 (2×CH₃), 27.47 (NHCH₂CH₂*CH*(CH₃)₂), 27.78 (NHCH₂*CH₂*CH₂NH₂), 38.46 (NHCH₂*CH₂*CH(CH₃)₂), 38.74 (NHCH₂CH₂*CH₂*NH₂), 41.93 (NH*CH₂*CH₂CH(CH₃)₂), 44.47 (NH*CH₂*CH₂CH₂NH₂), 94.44 (C-6), 96.34 (C-8), 118.60 (Ar-C), 124.10 (C-10), 124.50 (C-4), 125.30 (C-1), 131.02 (C-3), 131.45 (Ar-C), 131.92 (Ar-C), 132.99 (C-2), 133.71 (Ar-C), 134.64 (C-11), 149.53 (Ar-C), 152.57 (Ar-C), 157.49 (C-9), 158.95 (C-5) ppm. HRMS (FAB): found [M⁺] 389.23423; C₂₄H₂₉N₄O requires [M⁺]: 389.23437.

In an embodiment, the absorption and fluorescent emission of **ABP** in two solvents was determined. Electronic absorption and emission spectra of 10⁻⁶ and 10⁻⁷ M solutions in ethanol and water were measured for benzo[*a*]phenoxazinium chloride **ABP** (**Table 1**). The relative fluorescence quantum yields (*Φ*_{F}) were determined using Oxazine 1 as a standard (*Φ*_{F} = 0.11 in ethanol) at 570 nm excitation.

The absorption maxima (λ_{abs}) for compound **ABP** in ethanol is 614 nm, whereas in water λ_{abs} is 607 nm, with molar extinction coefficients (*ε*) higher, log *ε* is 4.93 and 4.85, being the lowest value in water. The emission maxima (λₑₘ) are 640 nm and 646 nm, in ethanol and water, respectively, at excitation of 570 nm, with moderate Stokes shifts (Δλ, 26 and 39 nm). As a result, the solvent change does not significantly modify the λ_{abs} values of **ABP,** with a hypochromic shift of 7 nm from ethanol to water. Comparison of the emission wavelength values in ethanol and water reveal a bathochromic shift of 6 nm when changing from alcohol to aqueous solution. Relative fluorescence quantum yield in ethanol is higher (0.39) than in water (0.21).

**Table 1 Photophysical data of PBP and ABP in ethanol, methanol and aqueous solution (C = 10⁻⁶ and 10⁻⁷ M). ^{a}Unit: nm.**

| | Ethanol | | | | | Methanol | | | | | Water | | | | |
|---|---|---|---|---|---|---|---|---|---|---|---|---|---|---|---|
| | λ_{abs}^{a} | log *ε* | λₑₘ^{a} | *Φ*_{F} | *Δλ*^{a} | λ_{abs}^{a} | log *ε* | λₑₘ^{a} | *Φ*_{F} | Δλ^{a} | λ_{abs}^{a} | log *ε* | λₑₘ^{a} | *Φ*_{F} | Δλ^{a} |
| **PBP** | 622 | 4.98 | 645 | 0.28 | 23 | 617 | 5.08 | 643 | 0.28 | 26 | 617 | 4.96 | 651 | 0.07 | 34 |
| **ABP** | 614 | 4.93 | 640 | 0.39 | 26 | -- | -- | -- | -- | -- | 607 | 4.85 | 646 | 0.21 | 39 |

### General experimental procedures

All melting points were measured on a Stuart SMP3 melting point apparatus. TLC analysis was carried out on 0.25 mm thick precoated silica plates (Merck Fertigplatten Kieselgel 60F₂₅₄) and spots were visualised under UV light. Chromatography on silica gel was carried out on Merck Kieselgel (230-240 mesh). IR spectra were determined on a BOMEM MB 104 spectrophotometer. UV-Vis absorption spectra (200 - 800 nm) were obtained using Shimadzu UV/3101PC spectrophotometer and fluorescence spectra with Spex Fluorolog spectrofluorometer. NMR spectra were obtained on a Varian Unity Plus Spectrometer at an operating frequency of 300 MHz for ¹H and 75.4 MHz for ¹³C or a Bruker Avance III 400 at an operating frequency of 400 MHz for ¹H and 100.6 MHz for ¹³C using the solvent peak as internal reference at 25 °C. All chemical shifts are given in ppm using *δ*_{H} Me₄Si = 0 ppm as reference and *J* values are given in Hz. Assignments were made by comparison of chemical shifts, peak multiplicities and *J* values and were supported by spin decoupling-double resonance and bidimensional heteronuclear correlation techniques. Low and high-resolution mass spectrometry analysis were performed at the "C.A.C.T.I. - Unidad de Espectrometria de Masas", at University of Vigo, Spain. Commercially available reagents were used as received.

### Method for preparation of the composition and application thereof

### Preparation of PBP or ABP formulation.

**PBP** (3.8×10⁻³ g, 9.96×10⁻⁶ mol) or **ABP** (3.8×10⁻³ g, 7.50×10⁻⁶ mol) was weighted in an analytical balance properly calibrated, and dissolved in ethanol (10 mL) previously measured in a graduated cylinder under stirring. The resulting alcoholic solution of **PBP** (0.04%) or **ABP** (0.04%) was used in the fixation and staining of cell samples.

### Procedure of fixation and staining samples with PBP formulation (smear) or ABP (0.04%) formulation (smear).

A sample is collected and placed on the slide as smear. Slide drying in the air (1 minute). The composition is applied in the slide (1 minute), and then washed with running water (5-15 seconds). Finally, observation and reading are carried out.

Procedure of fixation and staining samples with **PBP** formulation (smear and liquid environment).

The sample processing can be performed by smear or put the sample in fixative liquid and sent to the laboratory. In case it is sent to laboratory, it optionally and complementarily can proceed to the sample observation in smear with the following technique (optional). A sample is collected and placed on the slide as smear and in liquid environment. The sample is labelled and sends to laboratory. Slide drying in the air (1 minute). The composition is applied in the slide (1 minute), and afterword quickly washed with running water (5-15 seconds). Finally, observation and reading are carried out.

### Example(s) of application of the composition

Several laboratory tests were performed to demonstrate the usefulness and the innovation of the proposed disclosure. The dye composition proposed here was applied in 1200 gynecological cytology samples and in 800 oral cytology samples.

Each sample was stained by the new composition and the standard Papanicolaou staining in order to assess its colouring properties. The slides of the stained cytologies were analysed by experts in the field of cytological screening.

In these studies, it was verified that the different cellular structures were completely stained allowing their correct evaluation. This allows us to further state that this dye composition is compatible and ambivalent for gynecological and non-gynecological cytologies.

The compound presented has the necessary staining properties for the morphological analysis of the cells *in vitro.* The standard staining for cytology is Papanicolaou staining that uses various dyes and reagents to evidence cell structures. The invented composition consists of a completely new molecule which in an alcoholic-based mixture makes it possible to staining the cells with only a dye solution. The various steps of the Papanicolaou method are replaced by a single step of applying the composition. In just one minute it is possible to stain all the cellular structures reducing the times of staining and the costs with the same. The present disclosure eliminates staining artefacts by increasing diagnostic acuity.

**Figure 3** shows images of cervix cells where the fixative and staining composition was applied in the sample and after one minute, it was possible to see the cells.

**Table 2 shows images of cervix cells and oral cavity cells where the fixative and staining composition was applied; after one minute it was possible to see the cells.**

| Compound | | | | Solvent | Cell type | Result |
|---|---|---|---|---|---|---|
| Code | R | R₁ | R₂ | | | |
| **ABP** | (CH₂)₂CH(CH₃)₂ | (CH₂)₃NH₂·HBr | H | Methanol | Cervix | 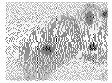 |
| | | | | Methanol/ Water (1:1) | | 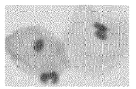 |
| | | | | Methanol | Oral cavity | 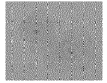 |
| **CBP**^{b} | (CH₂)₃Cl | (CH₂)₂CH₃ | (CH₂)₂CH₃ | Methanol | Cervix | 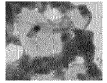 |
| | | | | Methanol/ Water (1:1) | | 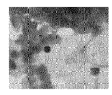 |
| | | | | Methanol | Oral cavity | 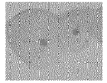 |
| **PBD**^{c} | (CH₂)₂CH₃ | (CH₂)₁₁CH₃ | H | Methanol | Cervix | 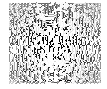 |
| | | | | Methanol/ Water (1:1) | | 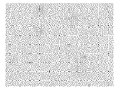 |
| | | | | Methanol | Oral cavity | 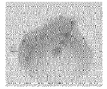 |
| **PBE** | (CH₂)₂CH₃ | (CH₂)₉CH₃ | H | Methanol | Cervix | 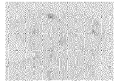 |
| | | | | Methanol/ Water (1:1) | | 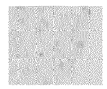 |
| | | | | Methanol | Oral cavity | 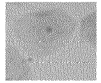 |

| | | | | | | |
|---|---|---|---|---|---|---|
| ^{b} Leitão M.I.P.S, Raju B. R, Sousa M. J., Coutinho P. J. G., GonçalvesM. S T. "Benzo[a]phenoxazinium chlorides functionalized with chloride atoms and/or ester groups. In Proceedings of the 19th Int. Electron. Conf. Synth. Org. Chem., Sciforum Electronic Conference Series, Vol. 19, 2015, a006. ^{c} Raju B. R., Naik S., Coutinho P.J. G., Gonçalves M. S. T. "Novel Nile Blue derivatives as fluorescent probes for DNA", Dyes Pigm. 99, 2013, 220-227. | | | | | | |

The disclosure should not be seen in any way restricted to the embodiments described and a person with ordinary skills in the art will foresee many possibilities to modifications thereof.

Furthermore, where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the disclosure, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

The above described embodiments are combinable. The following claims further set out particular embodiments of the disclosure.

## Claims

1. Compound comprising the following structure:

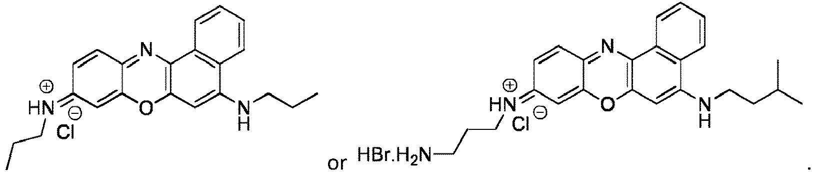

2. Compound according to the previous claim for use in a method of diagnosis in of neoplasia or infection in a sample.

3. Compound for use in a method of diagnosis of cancer or neoplasia in a sample comprising the following structure 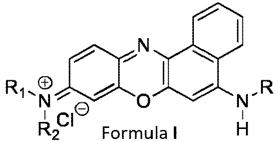
or another salt, solvate, hydrate, tautomer, stereoisomer,
wherein
R, R₁, R₂, are independently selected from each other;
R₁ and R₂ are selected from hydrogen, C₃-C₈ unsubstituted alkyl chain or (CH₂)₃NH₂.HBr; wherein R is a propyl group or an isopropyl group or an isopentyl group.

4. Compound for use according to any of claims 2 and 3, wherein R₁ or R₂ is a C₃ unsubstituted alkyl chain.

5. Compound for use according to any one of the claims 2 to 4, wherein R₁ or R₂ is propyl group or an isopropyl group, preferably wherein R₁ is propyl.

6. Compound for use according to any of the claims 2 to 5, wherein R₁ or R₂ is (CH₂)₃NH₂.HBr.

7. Compound for use according to any one of the claims 2 to 6, wherein R₂ is hydrogen.

8. Compound for use according to any of the claims 2 to 7, wherein the neoplasia is a cervix cancer or oral cavity cancer.

9. Composition for use in cell staining and fixation comprising the compound according to any one of the previous claims and a suitable solvent; preferably wherein the solvent is selected between water, alcohol base or their mixtures.

10. Composition for use according to the previous claim, wherein the concentration of compound is less than 0.1% (m/v).

11. Composition for use according to any one of the previous claims 9-10, wherein the concentration of compound varies between 0.02 - 0.1% (m/v), preferably wherein the quantity of compound is 0.02 - 0.075% (m/v), more preferably wherein the quantity of compound is 0.03 -0.04% (m/v).

12. Composition for use according to any one of the previous claims 9-11, wherein the alcohol base is selected between ethanol, methanol, isopropanol, or mixtures thereof; preferably wherein the alcohol base is ethanol,

13. Composition for use according to any one of the previous claims 9-12 for use in cells coloring and staining.

## Patentansprüche

1. Verbindung, umfassend die folgende Struktur:

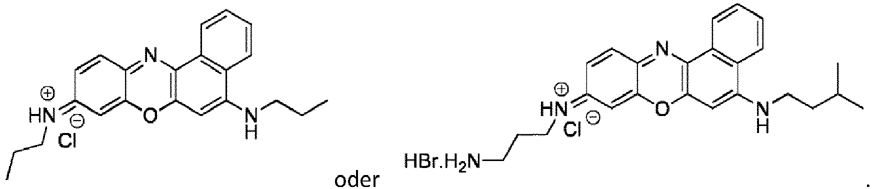

2. Verbindung nach dem vorangehenden Anspruch zur Verwendung in einem Verfahren zur Diagnose von Neoplasie oder einer Infektion in einer Probe.

3. Verbindung zur Verwendung in einem Verfahren zur Diagnose von Krebs oder Neoplasie in einer Probe, umfassend die folgende Struktur 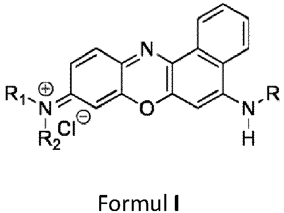
oder ein anderes Salz, Solvat, Hydrat, Tautomer oder Stereoisomer,
wobei
R, R₁, R₂ unabhängig voneinander ausgewählt werden;
R₁ und R₂ aus Wasserstoff, einer C₃-C₈ unsubstituierten Alkylkette oder (CH₂)₃NH₂.HBr ausgewählt sind;
wobei R eine Propylgruppe oder eine Isopropylgruppe oder eine Isopentylgruppe ist.

4. Verbindung zur Verwendung nach einem der Ansprüche 2 und 3, wobei R₁ oder R₂ eine C₃ unsubstituierte Alkylkette ist.

5. Verbindung zur Verwendung nach einem der Ansprüche 2 bis 4, wobei R₁ oder R₂ eine Propylgruppe oder eine Isopropylgruppe ist, wobei R₁ bevorzugt Propyl ist.

6. Verbindung zur Verwendung nach einem der Ansprüche 2 bis 5, wobei R₁ oder R₂ (CH₂)₃NH₂.HBr ist.

7. Verbindung zur Verwendung nach einem der Ansprüche 2 bis 6, wobei R₂ Wasserstoff ist.

8. Verbindung zur Verwendung nach einem der Ansprüche 2 bis 7, wobei die Neoplasie ein Zervixkarzinom oder ein Mundhöhlenkarzinom ist.

9. Zusammensetzung zur Verwendung bei der Färbung und Fixierung von Zellen, umfassend die Verbindung nach einem der vorangehenden Ansprüche und ein geeignetes Lösungsmittel; wobei das Lösungsmittel bevorzugt zwischen Wasser, einer Alkoholbasis oder deren Mischungen ausgewählt ist.

10. Zusammensetzung zur Verwendung nach dem vorangehenden Anspruch, wobei die Konzentration der Verbindung weniger als 0,1 % (m/v) beträgt.

11. Zusammensetzung zur Verwendung nach einem der vorangehenden Ansprüche 9-10, wobei die Konzentration der Verbindung zwischen 0,02 - 0,1% (m/v) variiert, wobei die Menge der Verbindung bevorzugt 0,02 - 0,075 % (m/v) beträgt; wobei die Menge der Verbindung besonders bevorzugt 0,03 - 0,04 % (m/v) beträgt.

12. Zusammensetzung zur Verwendung nach einem der vorangehenden Ansprüche 9-11, wobei die Alkoholbasis ausgewählt ist zwischen Ethanol, Methanol, Isopropanol oder Mischungen davon; wobei die Alkoholbasis bevorzugt Ethanol ist.

13. Zusammensetzung zur Verwendung nach einem der vorangehenden Ansprüche 9-12, zur Verwendung bei der Färbung und Anfärbung von Zellen.

## Revendications

1. Composé comprenant la structure suivante :

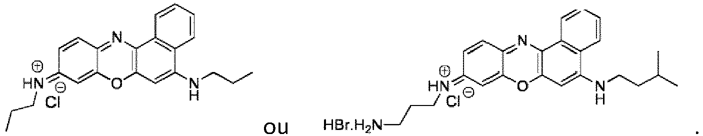

2. Composé selon la revendication précédente destiné à être utilisé dans un procédé de diagnostic de néoplasme ou d'infection dans un échantillon.

3. Composé destiné à être utilisé dans un procédé de diagnostic de cancer ou de néoplasme dans un échantillon comprenant la structure suivante 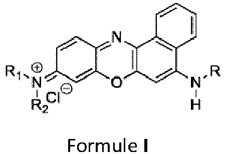
ou un autre sel, solvate, hydrate, tautomère, stéréoisomère,
dans lequel
R, R₁, R₂, sont choisis indépendamment parmi les uns et les autres ;
R₁ et R₂ sont choisis parmi l'hydrogène, la chaîne alkyle non substituée en Ca-Cs ou le (CH₂)₃NH₂.HBr;
dans lequel R est un groupe propyle ou un groupe isopropyle ou un groupe isopentyle.

4. Composé destiné à être utilisé selon l'une quelconque des revendications 2 et 3, dans lequel R₁ ou R₂ est une chaîne alkyle non substituée en C₃.

5. Composé destiné à être utilisé selon l'une quelconque des revendications 2 à 4, dans lequel R₁ ou R₂ est un groupe propyle ou un groupe isopropyle, préférablement dans lequel R₁ est du propyle.

6. Composé destiné à être utilisé selon l'une quelconque des revendications 2 à 5, dans lequel R₁ ou R₂ est le (CH₂)₃NH₂.HBr.

7. Composé destiné à être utilisé selon l'une quelconque des revendication 2 à 6, dans lequel R₂ est de l'hydrogène.

8. Composé destiné à être utilisé selon l'une quelconque des revendication 2 à 7, dans lequel le néoplasme est un cancer du col de l'utérus ou un cancer de la cavité buccale.

9. Composé destiné à être utilisé dans la coloration et fixation de cellules comprenant le composé selon l'une quelconque des revendications précédentes et un solvant adapté ; préférablement dans lequel le solvant est choisi parmi de l'eau, une base d'alcool ou des mélanges de ceux-ci.

10. Composé destiné à être utilisé selon la revendication précédente, dans lequel la concentration de composé est inférieure à 0,1% (m/v).

11. Composé destiné à être utilisé selon l'une quelconque des revendications précédentes 9-10, dans lequel la concentration du composé varie entre 0,02% et 0,1% (m/v), préférablement dans lequel la quantité de composé se situe entre 0,02% et 0,075% (m/v), plus préférablement dans lequel la quantité de composé se situe entre 0,03% et 0,04% (m/v).

12. Composé destiné à être utilisé selon l'une quelconque des revendications précédentes 9-11, dans lequel la base d'alcool est choisie parmi l'éthanol, le méthanol, l'isopropanol, ou des mélanges de ceux-ci ; préférablement dans lequel la base d'alcool est de l'éthanol.

13. Composé destiné à être utilisé selon l'une quelconque des revendications 9-12 pour une utilisation dans la coloration et la teinture de cellules.
